Europäisches Patentamt

European Patent Office

Office européen des brevets

(19)

(11) Publication number: **0 053 936**
**B1**

(12) **EUROPEAN PATENT SPECIFICATION**

(45) Date of publication of patent specification: **26.04.89**

(51) Int. Cl.⁴: **A 61 L 15/03**

(21) Application number: **81305764.3**

(22) Date of filing: **07.12.81**

(54) Surgical dressing.

(30) Priority: **08.12.80 GB 8039306**

(43) Date of publication of application:
**16.06.82 Bulletin 82/24**

(45) Publication of the grant of the patent:
**26.04.89 Bulletin 89/17**

(84) Designated Contracting States:
**AT DE FR GB IT**

(56) References cited:
**GB-A- 386 067**
**GB-A- 723 431**
**GB-A-1 301 101**
**The Condensed Chemical Dictionary, 9th ed.,**
**VAN NOSTRAND, Rheinhold Co., p. 163**
**Dictionnaire VIDAL, 58th ed., 1982, p. 239**
**MARTINDALE, 28th ed., Pharmaceutical Press,**
**1982, p. 79**
**The Lancet, 13 September 1980, p. 594**
**The Lancet, 2 February 1980, p. 271**

**The file contains technical information
submitted after the application was filed and
not included in this specification**

(73) Proprietor: **Johnson & Johnson Products Inc.**
**501 George Street**
**New Brunswick New Jersey 08903 (US)**

(72) Inventor: **Jackson, Stuart Windust**
**21B Claire Gardents Horndean**
**Portsmouth Hampshire (GB)**

(74) Representative: **Fisher, Adrian John et al**
**CARPMAELS & RANSFORD 43 Bloomsbury**
**Square**
**London WC1A 2RA (GB)**

(56) References cited:
**M.R. FROST et al., Microbiol. Letters, 1980, 13,
pp. 135-140**

**CHEMISTRY IN BRITAIN, vol. 16(3), March
1980, p. 172**

Courier Press, Leamington Spa, England.

## Description

This invention relates to surgical dressings.

Activated charcoal has long been known, and has often been used, for example in gas masks, for removing noxious chemicals from the air.

British Patent Specification No. 1,301,101 discloses an activated carbon cloth, produced by carbonising cellulosic cloth, e.g. a woven viscose rayon fabric, and activating the carbonised cloth. This cloth can sorb at least 50% of its own wieght of carbon tetrachloride, and has found medical use by virtue of its ability to remove offensive odours from heavily-infected wounds, such as varicose ulcers. (See, for example, the report by Butcher *et al* in Nursing Mirror, April 15, 1976, 64).

The odour-absorbing ability of wound dressings which incorporate a layer of activated carbon cloth is discussed in Chemistry in Britain, *16*(3), p.172 (1980) and in The Lancet, February 2, p. 271 (1980). The former reference states that such dressings generally improve the healing environment around a wound, while the latter suggests that the use of such dressings could possibly reduce the risk of infection, thus improving healing.

The Lancet, September 13, p. 594 (1980) discloses that charcoal cloth absorbs bacteria, but states that there is no conclusive evidence that it facilitates wound healing. It has not been suggested that activated carbon cloth is capable of killing bacteria, and nor have surgical dressings comprising the cloth been suggested for such use.

British Patent Specification No. 386,067, published as long ago as 1933, discloses activated carbonised fabric which is stated to kill or weaken bacteria. Also disclosed in this Patent Specification are surgical and like dressings constituted by the activated carbonised fabric or by an entangled mass of carbonised fibres. By the use of such carbonised fabric or entangled mass, the capillary action of the fibres is stated to be utilised to material advantage. The dressings are stated to be particularly suitable to act as supports for various chemical substances intended to act either therapeutically or aseptically. Thus, for example, it is stated that the dressings will hold, in considerable quantities, iodine, formol, lime, oxygen, bacillary toxines, and the like.

A known disadvantage of applying iodine to wounds in a highly labile form, for example as aqueous alcoholic solutions and the like, is that it discolours, irritates, and often injures skin and wounds when used for treating biologic tissues. It has been attempted to eliminate this disadvantage of iodine solutions by complexing iodine to water-soluble organic carriers such as polyvinylpyrrolidone and the like. Water-soluble complexes of this type, which are usually called iodophors, have been known for a long time, but presently available water-soluble iodophors have a tendency to release iodine too quickly. As a consequence, the disinfecting and sterilising effect of the water-soluble iodophor may cease very rapidly.

Attempts have been made to reduce the lability of the iodine in water-soluble iodophors by absorbing and/or adsorbing the iodophors to different soluble and insoluble materials such as metal salts, clay, talc etc. To the same end, efforts has been concentrated more recently on the development of insoluble iodophors. For example, British Patent Specification No. 2,031,441 discloses an insoluble complex of iodine with polypyrrolidone, and U.S. Specification No. 4,010,259 discloses insoluble complexes of iodine with various crosslinked polysaccharides.

We have now found that a surgical dressing of improved properties is obtained by providing an activated charcoal cloth of which the adsorptive sites are no more than 20% saturated with iodine or other antimicrobial agent. 20% saturation with iodine is usually equivalent to about 5% by weight of iodine on the cloth.

By binding the antimicrobial agent such as iodine to the adsorptive sites of the cloth, the above-described disadvantages of highly labile iodine are avoided. Morever, in contradistinction to the teachings of British Patent Specification No. 386,067, no reliance is placed on capillary action which, according to that Specification, is utilised to material advantage. Furthermore, our use of no more antimicrobial agent than is sufficient to saturate 20% of the adsorptive sites on the cloth (in contrast to the considerable quantities of antimicrobial agent mentioned in Specification No. 386,067) leaves at least 80% of the adsorptive sites available to adsorb microorganisms, e.g. bacteria, and also offensive odours.

According to the present invention, there is provided a surgical dressing comprising activated charcoal having an antimicrobial agent adsorbed thereto, the antimicrobial agent being present in an amount which saturates no more than 20% of the adsorptive sites which are capable of adsorbing said antimicrobial agent.

A surgical dressing in accordance with the present invention provides an agent in sufficient amount for killing micro-organism as well as being able to adsorb micro-organisms, e.g. bacteria, and odours. Forms of activated charcoal other than charcoal cloth, for example activated charcoal powder, may be impregnated and incorporated into surgical dressings in accordance with the present invention.

The anti-microbial agent may be, for example, a biocide such as a bacteriocide, a fungicide or a sporicide, or a bacteriostatic agent. Examples of anti-microbial agents which may be used in the present invention are iodine, chlorhexidine, bronopol, dibromopropamidine isethionate, chlorinated phenols, and antibiotics.

It will be understood that "surgical dressing" as used herein is intended to embrace all articles which are applied to the body, particularly with the intention of reducing or preventing infection, or absorbing body fluids. Included in the term "surgical dressings" are bandages, surgical sponges and packs, ward dressings of the kind sold under the Registered Trade Mark "STERIPAD", adhesive dressings, and such articles as tampons which may be used, for example, in the treatment of candidal vaginitis (vaginal thrush). The latter articles may, in accordance with the present invention, comprise activated charcoal impregnated with micronazole.

A great advantage of activated charcoal on which an anti-microbial agent has been adsorbed is that it may be applied to an infected wound, for example, without the antimicrobial agent being taken up systemically. This allows the use of powerful biocides, such as iodine, which might cause undesirable side effects if applied topically in considerable quantity.

It is particularly preferred tht the activated charcoal is in the form of an activated charcoal cloth.

Also provided by the present invention is a sheet or web of activated charcoal cloth, impregnated with an anti-microbial agent, for use in surgical dressings.

The anti-microbial agent is preferably iodine. Charcoal cloth may be impregnated with iodine in a variety of ways. For example, iodine may be sublimed in the presence of activated charcoal cloth, so that the iodine is adsorbed from the varpour phase. Activated charcoal cloth is available from Charcoal Cloth Limited, 21 Haviland Road, Ferndown Industrial Estate, Wimbourne, Dorset.

A preferred method of impregnating activated charcoal cloth comprises the step of immersing the cloth in a solution of elemental iodine in, for example, aqueous potassium iodide. By selecting the volume and concentration of the iodine solution used, and by immersing the cloth in the solution until the latter is decolourised, it is possible accurately to control the weight of iodine which is adsorbed onto the cloth.

Since it is essential to the present invention that the adsorptive sites of the activated charcoal cloth be no more than 20% saturated with iodine, it is necessary to know the weight of iodine which must be adsorbed onto a given amount of the cloth to achieve 100% saturation of the adsorptive sites. This may be determined as follows: A piece of the activated charcoal cloth is weighed and then immersed in a 0.1M solution of iodine in aqueous potassium iodide, until equilibrium is reached. Such equilibrium will usually have been reached within 5 minutes of immersing the cloth in the solution. If the solution becomes decolourised by the cloth before 5 minutes have elapsed, more iodine is added until the cloth is no longer able completely to decolourise the solution. The cloth is then removed from the solution, rinsed in deionised water and dried at room temperature. The dried cloth is then reweighed, and the weight of the adsorbed iodine calculated. This is the weight of iodine required to achieve 100% saturation of the absorptive sites of the piece of cloth tested.

It will be appreciated that the same procedure may be used to determine the amount of iodine required for 100% saturation of other forms of activated charcoal, and that analogous procedures will be applicable to measuring the amount of other anti-microbial agents required for 100% saturation.

The bacteriocidal properties of activated charcoal cloth, which had been impregnated to varying degrees with iodine, were measured in two different ways, referred to herein as the shaking test and the agar plate test.

For both tests, duplicate samples of charcoal cloth were impregnated to 0.1, 1, 5, 20 and 100% of saturation by immersion in iodine in aqueous potassium iodide and then washed and dried. 100% saturation required 0.0095 g of iodine per $cm^2$ of charcoal cloth. As a control, charcoal cloth itself was also tested for bacteriocidal properties.

The specimens of cloth were tested for their ability to kill *Escherichia coli*. An overnight culture in nutrient broth was prepared from a laboratory stock culture of the test organism. The bacteria from the overnight culture were washed three times with sterile distilled water by centrifugation at 3000 r.p.m. They were then resuspended in sterile distilled water to give a concentration of approximately $10^7$ orgs/ml.

Aliquots (10 ml) of the bacterial suspension were prepared in sterile screwcap bottles. Square pieces (7.5 × 7.5 cm) of each of the test specimens of cloth were introduced into the bottles of bacterial suspension. A count was performed on the suspension in each bottle (0 min), the bottles were shaken for 15 minutes on a mechanical wrist-action shaker and a second count was then made on the suspension in each bottle (15 min).

Each sample of cloth was removed aseptically from the suspension in contact with it, drained to remove excess moisture, and introduced into sterile distilled water (10 ml) contained in fresh sterile bottles. These bottles were then shaken for 15 minutes, after which counts were performed on the resulting suspensions (wash).

All bacterial counts were performed using sterile phosphate buffer pH 7.5 as diluent and the standard pour plate technique using nutrient agar.

For the agar plate test, each sample of cloth was then laid in contact with nutrient agar and the agar examined for bacterial growth after incubation at 37°C.

The results of these tests are shown in Table I.

## TABLE I

| Degree of impregnation | SHAKING TEST | | | AGAR TEST |
|---|---|---|---|---|
| | 0 min | 15 min | Wash | |
| 0% | $2 \times 10^7$ | $6 \times 10^5$ | $1.3 \times 10^5$ | Growth |
| 0.1% | $9 \times 10^6$ | $1 \times 10^3$ | $1 \times 10^1$ | " |
| | $3 \times 10^7$ | $6 \times 10^1$ | $1 \times 10^1$ | " |
| 1% | $2 \times 10^6$ | $5 \times 10^2$ | 0 | " |
| | $8 \times 10^6$ | $7 \times 10^2$ | $1 \times 10^1$ | " |
| 5% | $2 \times 10^7$ | 0 | 0 | No Growth |
| | $3 \times 10^7$ | $2 \times 10^2$ | 0 | " |
| 20% | $2 \times 10^7$ | $2 \times 10^1$ | 0 | " |
| | $5 \times 10^7$ | $8 \times 10^1$ | 0 | " |

It will be seen from Table I that the activated charcoal cloth itself is substantially non-biocidal. However, impregnation with even very small quantities of iodine is sufficient to improve greatly the performance of activated charcoal cloth in reducing the viable count of bacteria in aqueous suspensions. For example, even when only 5% of the maximum possible amount of iodine had been adsorbed, the number of bacteria remaining in suspension after shaking with the cloth was reduced by 5 or 7 orders of magnitude. Furthermore, no viable organisms could be found, on or washed from, the cloth. At 5% saturation with iodine, of course, 95% of the adsorptive sites on the charcoal cloth are still available to adsorb toxins or odiferous molecules.

It is known to use charcoal powder as a filler for foamed elastomeric resins, for use in carpet backings or insoles for shoes. Such a form of charcoal may be used in dressings according to the present invention but the charcoal must, of course, be impregnated with an antimicrobial agent to a saturation level of no more than 20%.

The present invention also provides a foamed elastomer comprising activated charcoal powder impregnated to no more than 20% saturation with an anti-microbial agent, for use in surgical dressings.

The activated charcoal powder may be impregnated with iodine or any other anti-microbial agent either before or after dispersal in the foam compound. A preferred method of impregnating the charcoal powder after dispersal in the foam compound is to diffuse the anti-microbial agent into the foam after the foam has been cured. This method is particularly advantageous if the foam compound used requires a relatively high temperature for curing, since such high temperatures might otherwise cause vaporisation or decomposition of the anti-microbial agent.

The charcoal powder may be dispersed in the foam compound before, during or after foaming.

The elastomeric based foam compound may be based upon a natural rubber latex, a styrene-butadiene rubber (SBR) latex or any other similar latex or mixture of these.

It is particularly advantageous that the charcoal powder or impregnated charcoal powder be present in an amount which is 5 to 50% of of the weight of the foamed compound and preferably 10 to 20%.

Providing always that an open cell foam is produced, or that the foam may by some suitable means be converted into an open cell foam, then it is possible to use activated charcoal powder as a filler in a foam produced by an extrusion foaming process. Examples of such foams are PVC and the class of foams known as polyurethanes.

The anti-microbial properties of a foamed elastomer comprising iodine-impregnated activated charcoal were examined by an *in vitro* test method, namely that of Thomas & Russell (F. Thomas & A. D. Russell Microbios Letters 1976, 2, 169—177).

Bacteria were cultured overnight in a nutrient broth and portions (0.2 ml.) were transferred to, and spread over, the surface of an agar medium which was either nutrient agar or 5% blood agar. On to this were then laid pieces (10 × 10 cm.) of the material under examination.

Four materials were tested, namely (1) Bactigras (tulle containing 0.5% chlorhexidine acetate in yellow soft paraffin) (2) Sofra-tulle (containing 1% framycetin sulphate in white soft paraffin and wool fat base) (3) SBR foam containing 6% charcoal with 5% adsorbed iodine (4) 1% chlorhexidine on filter-paper.

The results of the test are set out in Table II, from which it can be seen that the iodine-containing·SBR foam compares favourably with conventional wound dressings in respect of its activity against *Staph. aureus* in particular and also against the other three organisms tested.

## TABLE II

### Zone of Inhibition in mm

| Test Material | Staph. aureus | | E. coli | | Ps. aeruginosa | | S. cerevisiae Sabouraud | Growth under Dressing |
|---|---|---|---|---|---|---|---|---|
| | BA | NA | BA | NA | BA | NA | | |
| BACTIGRAS | 0.5* | 2* | 0.5* | 1* | 0* | 0* | 0* | + |
| SOFRA-TULLE | 0* | 3* | 0* | 2* | 0* | 0* | 0* | + |
| IODINE IMPREGNATED SBR FOAM | 5+ | 13 | 0 | 4 | 0 | 2 | 0 | - |
| 1% CHLORHEXIDINE FILTER PAPER CONTROL | 3 | 5 | 2 | 4 | 1 | 3 | 2.5 | - |

* Denote growth between weave of dressing. .

EP 0 053 936 B1

The anti-microbial properties of charcoal cloth with adsorbed iodine, and of foamed SBR comprising iodine-impegnated activated charcoal were also examined by an *in vivo* assay, the procedure for which was as follows:

Standard 5 cm² circular full-thickness excisions were made on the mid-dorsum of each of 60 anesthetized, clipped male 500 g Hartley strain guinea pigs supplied by Charles River Laboratories, New Springville, New York. The procedure was performed on two sets of 30 guinea pigs each, with each set including a standard control group of 5 guinea pigs whose wounds were treated with an occlusive polyvinylidene chloride film (SARANWRAP, The Dow Chemical Company, Indianapolis, Indiana) attached to a DERMICEL Taffeta Tape backing to prevent wrinkling of the dressing. All experimental dressings were also covered with these standard occlusive dressings to prevent drying of the wound, and the combined dressing was held in place with ELASTIKON Elastic Tape as an exterior wrapping on all 60 animals. This occlusive dressing served the dual purpose of permitting optimal repair since air exposed excisions contract less than half as rapidly as occluded excisions, and also of permitting maximal proliferation of micro-organisms in the wound bed.

Wound on 5 guinea pigs were treated with each experimental dressing immediately after wounding. The experimental dressing extended beyond the edges of the 25 cm² circular occlusive dressing, and was held in place by wound clipping it to the exterior ELASTIKON wrapping.

Auxiliary observations recorded on the eighth day after wounding when the animals were sacrificed, included presence or absence of wound rim edema and undermining and focal erosions or sparsity of granulation tissue within the wound bed.

Wound surface micro-organism were sampled by surface swabbing with CALGISWABS on day 4 after wounding. Each swab was dispersed by vortexing into 10 ml of 1 percent sodium citrate. Serial dilutions were made and plated on Trypticase Soy Agar. After the plates were incubated for 48 hours at 35°C. The colony forming units were counted. A hundredfold or greater reduction in wound surface colony forming units samples from an experimentally dressed wound compared with the same-study occluded control wounds was deemed a significant anti-bacterial effect.

The results of this test are shown in Table III for four different dressings, namely

A) a soft weave fabric control,

B) charcoal cloth with iodine adsorbed to 20% saturation,

C) polycoated KEYBACK wound dressing covered with SBR foam containing activated charcoal with 20% adsorbed iodine,

D) polycoated KEYBACK wound dressing, covered with SBR foam with activated charcoal filler, the filled foam having ben treated with iodine solution to 20% saturation.

TABLE III

| Dressing | Number of wounds showing significant anti-bacterial effect after 4 days/Total |
|---|---|
| A | 0/5 |
| B | 3/5 |
| C | 2/5 |
| D | 3/5 |

The surgical dressing of the present invention may be of any suitable form. Many conventional designs of dressing are envisaged as being suitable for the practice of the present invention. Examples of suitable designs are now described with reference to the accompanying drawings, in which:

Figure 1 is a plan view of a surgical dressing in accordance with the invention,

Figure 2 is a schematic section on A—A of Figure 1,

Figure 3 is a schematic cross-sectional view of a second surgical dressing in accordance with the invention,

Figure 4 is a schematic cross-sectional view of a third dressing, and

Figure 5 is a schematic cross-sectional view of an adhesive dressing which incorporates the dressing of Figure 4.

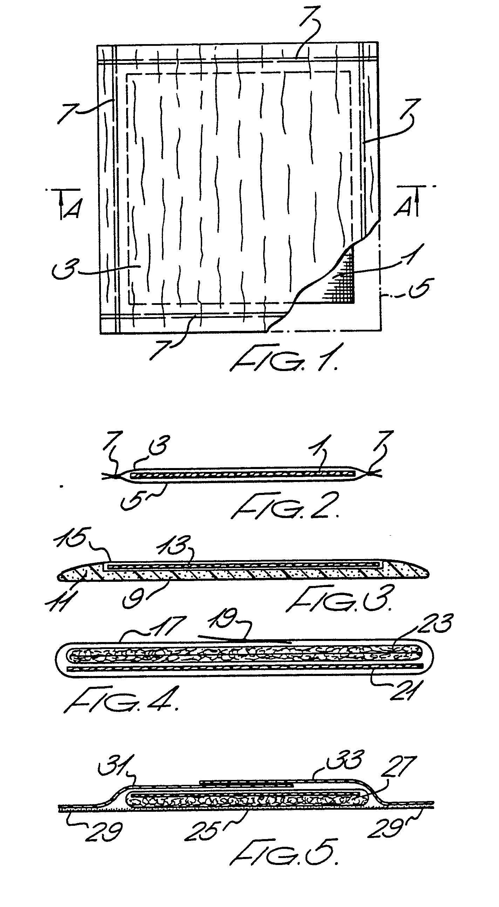

Referring to Figures 1 and 2, a simple dressing according to the present invention comprises a square of iodine-impregnated charcoal cloth 1, sandwiched between two larger squares 3,5 of spun-bonded porous nylon fabric. The squares 3,5 are fastened to each other by heat-welding along lines 7 which are formed a short distance in from each edge of the squares. By this means the charcoal cloth 1 is fully

7

enclosed within a pouch of porous nylon fabric. A dressing of this sort may conveniently sterilised b gamma irradiation and packaged within a sterile, waterproof and hermetically-sealed envelope.

Figure 3 illustrates an alternative construction of dressing, comprising a backing sheet 9 or foame elastomeric resin. The edges 11 of the backing sheet are thickened so as to form a shallow well in which piece of impregnated charcoal cloth 13 is positioned. The cloth 13 is covered by a highly porous fabric laye 15, for example an apertured non-woven web, which is attached to the edges 11 of the backing sheet 9 b adhesive or by welding. The dressing may be made as a continuous strip in which the fabric layer i attached to the backing sheet only by the longitudinal edges. Such a strip may then be cut to the desire size. Alternatively, the dressing may be made in pre-formed squares or rectangles, in which case it i desirable that the fabric layer is attached to the backing sheet by all four edges.

Figure 4 shows an alternative form of dressing which may be made in continuous lengths. A strip 17 c highly porous fabric fabric is formed into a sleeve by folding the longitudinal edges over and fastenin them together by a line or adhesive 19. The sleeve so formed contains a strip 21 of impregnated charco cloth, and a thicker strip of highly absorbent lint 23. The lint serves to absorb fluids which are exuded by th wound over which the dressing is placed.

In some applications it will be convenient to incorporate impregnated charcoal into a convention sticking plaster. Figure 5 illustrates a sticking plaster of this type. The sticking plaster comprises a adhesive-coated backing sheet 25 of plastics material which is preferably perforated. In the central portio of the backing sheet 25 is placed an absorbent pad 27, such as a short length taken from the continuou dressing illustrated in Figure 4. The absorbent pad 27 is protected from becoming soiled before use b removable, overlapping plastics films 31, 33 which are held in place by the adhesive regions 29 round th edge of the backing sheet 25. In use, the films 31, 33 are peeled from the backing sheet, thus exposing th adhesive regions 29, in the manner of a conventional sticking plaster. The plaster is then ready fo application to the patient.

It will be appreciated that it is also possible simply to apply, for example, iodine-impregnated activate charcoal cloth or an elastomeric foam pad filled with iodine-impregnated activated charcoal, to a woun and to hold it in position by means of a conventional bandage or adhesive tape.

Preferably, the dressings are applied to the patient so as to bring the impregnated charcoal as near a possible to the infected site. If, however, impregnated charcoal cloth is to be used, it is desirable that a least one layer of fabric is interposed between the infected site and the impregnated cloth, since the latte has a slight tendency to produce free particles of charcoal, which might cause distress to the patient deposited on the wound or surrounding skin. Ideally, the fabric used is a specialist non-adherent woun contact layer, such as N—A (Trade Mark) Dressing, which may be removed from the majority of wound without trauma.

Each embodiment of surgical dressing described above with reference to the Drawings comprises a envelope or sleeve containing the activated charcoal cloth, the envelope or sleeve defining a facing laye for contacting the wound. The activated charcoal cloth may be impregnated with varying amounts of anti microbial agent, especially iodine, up to a maximum of 20% saturation. For example, the degree c saturation may be as described above with reference to Table I, depending on the end-use of the dressing

## Claims

1. A surgical dressing comprising activated charcoal an anti-microbial agent adsorbed thereto, the anti microbial agent being present in an amount which saturates no more than 20% of the adsorptive site which are cable of adsorbing said anti-microbial agent.

2. A dressing according to claim 1 wherein the anti-microbial agent is present in an amount sufficient t saturate no more than 5% of said adsorptive sites.

3. A dressing according to claim 1 or claim 2 wherein said activated charcoal is in the form of charco cloth.

4. A dressing according to claim 1 or claim 2 wherein said activated charcoal is in the form of powdere charcoal distributed in a foamed elastomeric resin.

5. A dressing according to any preceding claim wherein said anti-microbial agent is iodine.

6. A tampon comprising activated charcoal having miconazole absorbed thereto, the miconazole bein present in an amount sufficient to saturate no more than 20% of the adsorptive sites which are capable c adsorbing the miconazole.

7. A sheet or web of activated charcoal cloth having an anti-microbial agent adsorbed thereto, the anti microbial agent being present in an amount sufficient to saturate no more than 20% of the adsorptive site which are capable of adsorbing said anti-microbial agent.

8. A method of making a sheet or web of activated charcoal according to claim 7 comprising the step c immersing said cloth in a solution of elemental iodine in aqueous potassium iodide.

9. A foamed elastomer comprising activated charcoal powder having an anti-microbial adsorbe thereto, the anti-microbial agent being present in an amount sufficient to saturate no more than 20% of th adsorptive sites which are capable of adsorbing said anti-microbial agent.

10. A method of making a foamed elastomer according to claim 9 comprising the step of diffusing th anti-microbial agent into the foam and curing the foam.

# EP 0 053 936 B1

## Patentansprüche

1. Chirurgischer Verband mit einem Gehalt an Aktivkohle, an der ein antimikrobielles Mittel adsorbiert ist, wobei das antimikrobielle Mittel in einer Menge voliegt, welche nicht mehr als 20% der adsorbierenden Stellen, die zur Adsorption des genannten antimikrobiellen Mittels befähigt sind, absättigt.

2. Verband nach Anspruch 1, worin das antimikrobielle Mittel in einer Menge vorliegt, die zur Sättigung von nicht mehr als 5% der genannten adsorbierenden Stellen ausreicht.

3. Verband nach Anspruch 1 oder Anspruch 2, worin die genannte Aktivkohle in der Form von Aktivkohle-Stoff vorliegt.

4. Verband nach Anspruch 1 oder Anspruch 2, worin die genannte Aktivkohle in der Form von pulverförmiger Aktivkohle vorliegt, die in einem geschäumten Elastomerharz verteilt ist.

5. Verband nach einem der vorhergehenden Ansprüche, worin das genannte antimikrobielle Mittel Jod ist.

6. Tampon mit einem Gehalt an Aktivkohle, an der Miconazol adsorbiert ist, wobei das Miconazol in einer Mwnge vorliegt, die zur Sättigung von nicht mehr als 20% der adsorbierenden Stellen, die zur Adsorption von Miconazol befähigt sind, ausreicht.

7. Blatt oder Bahn aus Aktivkohle-Stoff, an dem ein antimikrobielles Mittel adsorbiert ist, wobei das antimikrobielle Mittel in einer Menge vorliegt, die zur Sättigung von nicht mehr als 20% der adsorbierenden Stellen, die zur Adsorption des genannten antimikrobiellen Mittels befähigt sind, ausreicht.

8. Verfahren zur Herstellung eines Blattes oder einer Bahn aus Aktivkohle-Stoff nach Anspruch 7, welches die Stufe des Eintauchens des genannten Stoffes in eine Lösung von elementarem Jod in wässrigem Kaliumjodid umfaßt.

9. Geschäumtes Elastomer mit einem Gehalt an Aktivekohlepulver, an dem ein antimikrobielles Mittel adsorbiert ist, wobei das antimikrobielle Mittel in einer Menge vorliegt, die zur Sättigung von nicht mehr als 20% der adsorbierenden Stellen, die zur Adsorption des genannten antimikrobiellen Mittels befähigt sind, ausreicht.

10. Verfahren zur Herstellung eines geschäumten Elastomers nach Anspruch 9, welches die Stufe des Eindiffundierens des antimikrobiellen Mittels in den Schaum und das Härten des Schaumes umfaßt.

## Revendications

1. Pansement chirurgical comprenant du charbon activé sur lequel est adsorbé un agent anti-microbien, l'agent anti-microbien étant présent en une quantité qui sature au plu 20% des sites d'adsorption qui sont capables d'adsorber ledit agent anti-microbien.

2. Pansement selon la revendication 1 dans lequel l'agent anti-microbien est présent en une quantité suffisante pour saturer au plus 5% desdits sites d'adsorption.

3. Pansement selon la revendication 1 ou la revendication 2 dans lequel ledit charbon activé est sous forme de toile de charbon.

4. Pansement selon la revendication 1 ou la revendication 2 dans lequel ledit charbon activé est sous forme de charbon en poudre réparti dans une résine élastomérique en mousse.

5. Pansement selon l'une quelconque des revendications précédentes dans lequel ledit agent anti-microbien est l'iodide.

6. Tampon comprenant du charbon activé sur lequel est adsorbé du miconazole, le miconazole étant présent en une quantité suffisante pour saturer au plus 20% des sites d'adsorption qui sont capables d'adsorber le miconazole.

7. Feuille ou voile de toile de charbon activé sur laquelle est adsorbé un agent anti-microbien, l'agent anti-microbien étant présent en une quantité suffisante pour saturer au plus 20% des sites d'adsorption qui sont capables d'adsorber ledit agent anti-microbien.

8. Procédé de fabrication d'une feuille ou voile de toile de charbon activé selon la revendication 7 comprenant l'étape d'immersion de ladite toile dans une solution d'iode élémentaire dans l'iodure de potassium aqueux.

9. Elastomère en mousse comprenant une poudre de charbon activé sur laquelle est adsorbé un agent anti-microbien, l'agent anti-microbien étant présent en une quantité suffisante pour saturer au plus 20% des sites d'adsorption qui sont capables d'adsorber ledit agent anti-microbien.

10. Procédé de préparation d'un élastomère en mousse selon la revendication 9 comprenant l'étape de diffusion de l'agent anti-microbien dans la mousse et de réticulation de la mousse.

FIG. 1.

FIG. 2.

FIG. 3.

FIG. 4.

FIG. 5.